Europäisches Patentamt

European Patent Office

Office européen des brevets

(19)

(11) Numéro de publication : **0 452 240 A1**

(12)

# DEMANDE DE BREVET EUROPEEN

(21) Numéro de dépôt : **91460022.6**

(22) Date de dépôt : **11.04.91**

(51) Int. Cl.⁵ : **C12M 1/12, C12P 7/52**

(30) Priorité : **13.04.90 FR 9004985**

(43) Date de publication de la demande :
**16.10.91 Bulletin 91/42**

(84) Etats contractants désignés :
**AT BE CH DE DK ES FR GB IT LI NL**

(71) Demandeur : **BRITECH S.A.**
**12 rue de Rennes**
**F-35220 Chateaubourg (FR)**

(72) Inventeur : **Boyaval, Patrick**
**6 allée d'Elven**
**F-35700 Rennes (FR)**
Inventeur : **Colomban, Agnès**
**10 rue Nationale**
**F-35300 Fougères (FR)**
Inventeur : **Roger, Loic**
**10 rue Paul Verlaine**
**F-35135 Chantepie (FR)**

(74) Mandataire : **Corlau, Vincent**
**c/o Cabinet Vidon Immeuble Germanium 80**
**avenue des Buttes de Coesmes**
**F-35700 Rennes (FR)**

(54) **Procédé et dispositif de fermentation semi-continue, notamment pour la fabrication industrielle d'un mélange biologique à base d'acide propionique.**

(57)     Le domaine de l'invention est celui de la fermentation semi-continue d'une source carbonée pour la fabrication d'un mélange biologique à base d'acide propionique, selon un processus industrialisable.

Le dispositif selon l'invention comprend avantageusement une cuve de fermentation (6) comportant deux boucles externes (9,19) de circulation du substrat fermenté :

— une boucle (9) de brassage du substrat fermenté effectuant un prélèvement et une réintroduction du substrat fermenté dans la cuve (6) de fermentation,

— une boucle (19) d'extraction du mélange (21) biologique à base d'acide propionique fabriqué (21), incluant un module (22) de séparation mélange biologique fabriqué (21)/bactéries (23).

Le procédé mis en oeuvre est du type semi-continu, formé d'une succession de cycles de fermentation/extraction avec recyclage intégral de la biomasse, puis recyclage partiel après obtention d'une concentration en biomasse prédéterminée.

EP 0 452 240 A1

Fig. 1

Le domaine de l'invention est celui de la fermentation semi-continue d'une source carbonée pour la fabrication d'un mélange biologique à base d'acide propionique.

Plus précisément, la présente invention concerne un procédé et dispositif de fermentation d'un substrat à l'aide de bactéries propioniques pour la fabrication d'acide propionique.

L'acide propionique et ses sels sont connus pour leur propriétés antifongiques. Ils sont notamment employés en panification et biscuiterie. Ils sont en partie à l'origine du goût de fromages à pâte cuite pressée du type Emmental.

Dans la plupart des pays c'est l'acide propionique ou ses sels produits par voie chimique qui, en temps qu'additifs, entrent dans la composition des aliments. Or, la législation en vigueur dans certains pays interdit l'utilisation de produits synthétiques pour la fabrication de denrées alimentaires destinées à l'homme. De plus, une masse croissante de consommateurs tend à préférer les produits d'origine naturelle entrant dans la composition d'aliments aux produits synthétiques. Il est alors intéressant de proposer des substituts d'origine biologique.

L'acide propionique biologique peut être incorporé dans les aliments par l'intermédiaire d'un produit fermenté par des bactéries propioniques.

La fermentation effectuée produit également, en moindre quantité, de l'acide acétique qui constitue un agent conservateur polyvalent.

Le produit naturel, ajouté à une préparation fromagère, a ainsi la double fonction de base aromatique et de conservateur et permet de réduire le temps de production et plus particulièrement le temps d'affinage, dont le rôle est notamment de permettre l'apparition des arômes.

On connait trois méthodes de fermentation applicables notamment à la fabrication d'acide propionique biologique : fermentation par lot, fermentation continue, fermentation semi-continue. Toutefois aucune de ces méthodes n'est actuellement mise en oeuvre industriellement en fermentation propionique, pour les raisons présentées ci-après.

La fermentation par lot, encore appelée fermentation "batch", consiste à introduire dans une cuve de fermentation un substrat à fermenter et un inoculum composé de bactéries. Après fermentation du substrat, ce qui correspond par exemple à une fin de période de reproduction des bactéries, la totalité du produit fermenté est extraite de la cuve de fermentation et les bactéries sont séparées du produit à obtenir. Le procédé peut alors reprendre par l'introduction d'un substrat et d'un inoculum neufs dans la cuve de fermentation. Les principaux avantages de la fermentation par lot sont la simplicité de la mise en oeuvre du dispositif et l'obtention d'une importante quantité de produit par une seule opération de séparation. L'inconvénient majeur de la fermentation par lot est qu'elle nécessite un temps de fermentation très long, particulièrement en fermentation propionique.

Une autre méthode de fermentation est la fermentation continue. Cette méthode consiste à extraire de façon continue de la cuve de fermentation la substance fermentée, à des fins de séparation du produit à obtenir des bactéries, et à rajouter continûment au milieu de fermentation une quantité de substrat neuf de telle sorte que la cuve contienne toujours le même volume de substance. L'avantage de cette méthode de fermentation est que l'on obtient de façon continue le produit fabriqué. Cependant, la fermentation continue est encore très difficile à maîtriser à l'échelle industrielle.

Par ailleurs, lorsque l'extraction du produit à obtenir des bactéries est effectuée par filtration, la mise en oeuvre de tels procédés implique la présence de deux modules de filtration, afin de permettre le nettoyage d'un filtre pendant que l'autre est utilisé, pour ne pas avoir à interrompre l'opération de fermentation. Cet inconvénient est majeur du fait du coût de chaque filtre à l'unité.

Des procédés de fermentation continue sont notamment décrits dans le document de brevet US n° 3.067.107 de WAYMAN et al. et dans la demande PCT n° 85/01064 de CHERYAN et al.

La troisième principale méthode de fermentation est la fermentation semi-continue. Cette méthode consiste à réaliser des cycles successifs de fermentation au cours desquels on retire périodiquement de la cuve de fermentation au moins une partie du milieu fermenté, on en extrait le produit fabriqué, et on introduit dans la cuve de fermentation un volume de substrat neuf correspondant au volume précédemment prélevé. Le cas échéant, on peut inclure les bactéries dans des gels, les absorber sur des supports poreux ou pulvérulents tel que la chaux le Kieselguhr ou le carbonate de calcium.

Un procédé de fermentation semi-continue d'acide propionique est notamment décrit dans le document de brevet US n° 2.020.251 de STILES. Ce document propose d'utiliser un substrat constitué de mélasses, d'alumine activée et de bentonite. La fermentation semi-continue est effectuée en remplaçant 50 % de la matière fermentée prélevée par un volume équivalent de substrat neuf. Cependant, le temps de fermentation est très long, en l'occurence 7 jours, bien que l'alumine activée et la bentonite jouent le rôle de catalyseurs.

Un autre document concernant la fermentation semi-continue d'acide propionique est le brevet US n° 1.932.755 de STILES et al. qui propose d'ajouter des bactéries lactiques aux bactéries propioniques, afin d'accélérer la vitesse de fermentation. Cependant, la fermentation est là aussi très longue et il n'est pas envisageable de produire de l'acide propionique en grande quantité avec un tel procédé.

Les articles concernant la fabrication d'acide propionique (Applied an Environmental Microbiology, Feb. 1986, p.427 - 428, vol. 51 n°2 ; Applied Microbiology and Biotechnology, 1987, 25 : p. 434 - 437; Journal of Applied Bacteriology 1989, 66, p. 175 - 184) mentionnent tous l'inconvénient dirimant de la faible vitesse de reproduction des bactéries propioniques.

La compétitivité industrielle de la fermentation propionique est faible notamment à cause:
– du faible taux de multiplication des *Propionibacterium* (bactéries propioniques),
– de la faible concentration cellulaire atteinte à la fin d'un cycle de croissance normal,
– du long temps de latence,
– de la faible résistance à la contamination bactérienne par des souches plus reproductives.

C'est pourquoi les procédés connus proposent notamment d'employer d'autres souches, d'ajouter des agents catalytiques ou de réaliser des co-cultures avec des bactéries lactiques pour stimuler le métabolisme des bactéries propioniques.

Les procédés existants ne répondent donc pas aux critères d'une fabrication d'acide propionique biologique en grande quantité, dans des conditions industrielles, à partir d'une biomasse exclusivement constituée de bactéries propioniques.

La présente invention a notamment pour objectif de pallier ces inconvénients.

Plus précisément, un premier objectif de l'invention est de mettre en oeuvre un procédé et dispositif de fermentation d'acide propionique exploitable industriellement.

Un autre objectif de l'invention est de fournir un tel procédé et un tel dispositif permettant de pallier les divers inconvénients et limitations de la fermentation propionique tels que la faible vitesse de reproduction et la sensibilité à la contamination du milieu de culture par des bactéries concurrentes.

Un objectif complémentaire de l'invention est de mettre en oeuvre un dispositif de fermentation facilitant la prise de mesures (pH, température,...) du milieu de fermentation.

Un autre objectif de l'invention est d'augmenter la concentration cellulaire en vue d'accroître la productivité volumique c'est à dire la quantité d'acide propionique par unité de volume et par unité de temps, sans utilisation de support pour les bactéries.

Ces objectifs, ainsi que d'autres qui apparaîtront par la suite, sont atteints grâce à un procédé de fermentation semi-continue d'un milieu de culture comprenant une source carbonée, notamment une substance à base de lactosérum, pour la fabrication industrielle d'un mélange biologique à base d'acide propionique à l'aide d'une biomasse produite à partir d'un inoculum de bactéries du type *Propionibacterium*, caractérisé en ce qu'il consiste à réaliser une succession de cycles de fermentation/extraction, constitués chacun :
– d'une première phase de fermentation au cours de laquelle ladite biomasse interagit en milieu fermé avec une nouvelle quantité de ladite source carbonée ;
– d'une seconde phase d'extraction périodique dudit mélange biologique fabriqué, et de recyclage de la biomasse développée à partir de l'inoculum pour la fabrication de mélange biologique au cours de la phase de fermentation du cycle suivant, et comprenant :
– une première période formée d'une première succession de cycles fermentation/extraction, destinée à assurer la croissance de la biomasse constituée par lesdites bactéries du type *Propionibacterium* jusqu'à obtenir une quantité de biomasse prédéterminée ($X_i$), la totalité de la biomasse étant recyclée d'un cycle à l'autre,
– une seconde période formée d'une seconde succession de cycles fermentation/extraction, réalisés en maintenant ladite quantité de biomasse autour de ladite quantité prédéterminée ($X_i$), en ne recyclant qu'une portion correspondante de la biomasse.

Dans un mode de réalisation préférentiel, le passage de ladite phase de fermentation à ladite phase d'extraction est effectué de façon à optimiser l'un des critères appartenant au groupe comprenant la maximisation du rendement de fabrication dudit mélange biologique à base d'acide propionique, et la maximisation de la vitesse de croissance desdites bactéries du type *Propionibacterium*.

Avantageusement, le passage de ladite phase de fermentation à ladite phase d'extraction est effectué lors de la réalisation d'au moins l'un des critères suivants:
– la teneur résiduelle en source carbonée est inférieure à 10 %, préférentiellement 5 % de la quantité de source carbonée introduite ;
– la concentration en acide propionique produit dépasse un seuil de 40 % environ de la concentration initiale en source carbonée.

Le dépassement du seuil de concentration en acide est préférentiellement détecté à partir de la surveillance de la vitesse d'ajout d'un produit neutralisant au mélange biologique pour maintenir le pH.

Selon l'invention, avantageusement, la concentration en bactéries et/ou la concentration en acide acétique et propionique, dans le milieu de fermentation, dépasse un seuil antibactérien prédéterminé au moins au cours d'une portion de la phase d'extraction et/ou d'une portion de la phase de fermentation d'au moins un desdits cycles.

L'invention concerne également un dispositif adapté à un tel procédé, constitué d'une cuve de fermentation comportant deux boucles externes de circulation du substrat fermenté:

– une boucle de brassage du substrat fermenté effectuant un prélèvement et une réintroduction dudit substrat fermenté dans ladite cuve de fermentation,

– une boucle d'extraction dudit mélange biologique à base d'acide propionique fabriqué, incluant un module de séparation mélange biologique fabriqué/bactéries,

et comprend des moyens d'aiguillage sélectif de la circulation dudit substrat fermenté alternativement dans l'une et l'autre desdites boucles pilotés en fonction d'un processus de fermentation semi-continue.

Le procédé selon la présente invention peut aussi avantageusement être utilisé pour la fabrication de biomasse, en quantité importante et pendant un temps optimisé, par exemple en ne mettant en oeuvre que la première des deux périodes du procédé. On notera de plus que le fonctionnement du procédé au cours de la seconde période permet de récuperer à chaque cycle une quantité de biomasse excédentaire, qui peut ensuite être exploitée indépendamment.

D'autres caractéristiques et avantages de la présente invention apparaîtront à la lecture de la description suivante d'un mode de mise en oeuvre préférentiel de l'invention, donné à titre illustratif et non limitatif, et des figures annexées dans lesquelles :

– la figure 1 représente un mode de réalisation préférentiel du dispositif selon la présente invention.

– les figures 2A et 2B représentent les évolutions de concentration de biomasse et de production d'acide propionique relevées dans un prototype du dispositif de la figure 1 pendant une période de 250 heures permettant d'atteindre une quantité de biomasse prédéterminée $X_f$.

– la figure 3 représente l'évolution de la croissance d'un contaminant microbien suivant la concentration d'acide propionique dans un milieu de fermentation de façon à illustrer le principe de lutte antibactérienne mis en oeuvre dans l'invention.

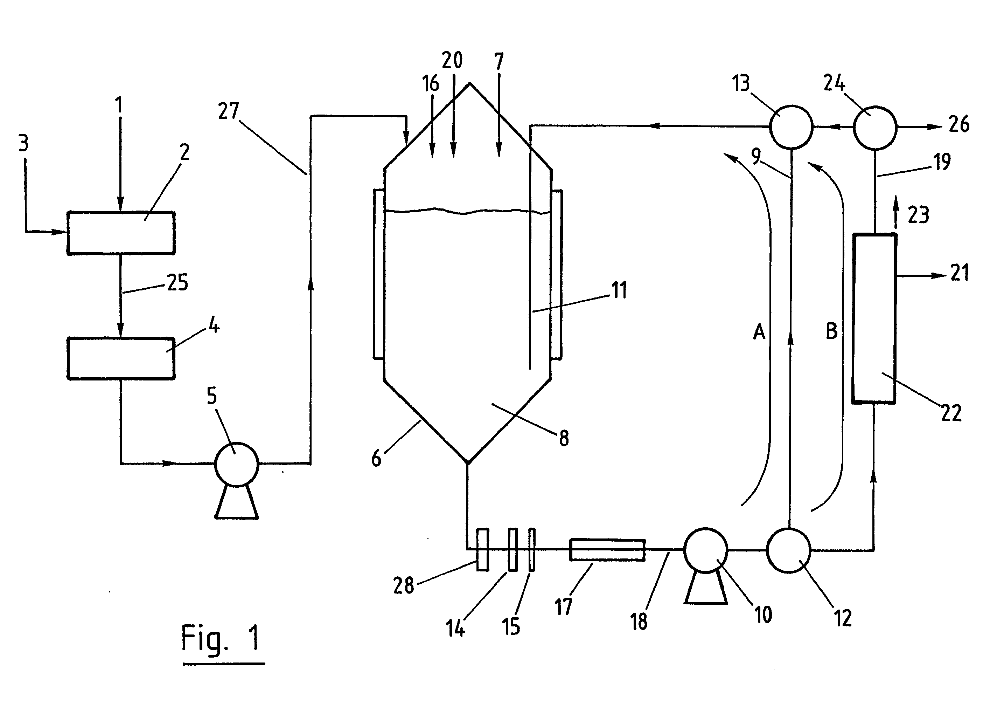

La figure 1 représente un mode de mise en oeuvre préférentiel du dispositif selon la présente invention pour la fabrication d'un mélange biologique à base d'acide propionique. Une source carbonée déprotéinée 1 est introduite dans un module 2 d'enrichissement avec un complément azoté 3. Le milieu de culture 25 résultant est stérilisé 4 puis introduit dans une cuve de fermentation 6 grâce à une pompe 5. La cuve 6 de fermentation est avantageusement une cuve à fond conique, calorifugée et ne comportant pas d'agitateur rotatif.

La cuve 6 comporte des moyens d'alimentation en inoculum 7 constitué de bactéries propioniques. Afin de travailler en légère surpression et de se rapprocher des conditions d'anaérobie, de l'azote 20 est avantageusement insufflé dans le dôme de la cuve 6 de fermentation.

La cuve 6 comporte également des moyens 16 d'introduction d'un neutralisant destiné à maintenir constant le pH du milieu 8 de fermentation.

La fermentation commence lorsque l'inoculum 7 est rajouté au milieu 27 introduit dans la cuve 6.

Le substrat fermenté 8 présent dans la cuve 6 de fermentation est constamment brassé grâce à une première boucle 9 externe, appelée boucle de brassage 9. Deux vannes 12, 13 permettent une circulation continue du produit retiré suivant le chemin A. Une partie du substrat fermenté 8 présent dans la cuve 6 est continuellement pompée 10 de la partie inférieure de la cuve 6 vers la boucle de brassage 9 et réintroduite dans le milieu de fermentation. En vue d'éviter toute production excessive de mousse, le milieu retourne dans le fermenteur 6 par une canne 11 plongeante sous le niveau du milieu de fermentation.

Cette circulation assure un brassage du milieu de fermentation. La présence d'une boucle de brassage 9 permet de diminuer les coûts de lutte contre les risques de contamination d'un brassage encourus soit lors d'une intervention en cours de fonctionnement, soit due à la présence d'un agitateur difficile à nettoyer. De plus, un agitateur est relativement onéreux et pose des problèmes d'étanchéité.

La boucle de brassage 9 permet également l'installation de capteurs de de pH 14, de température 15, et de teneur en biomasse 28 à l'extérieur de la cuve 6 sur la partie 18.

Le pH est maintenu à environ 6,5 par ajout de $NH_4$ 16 gazeux ou liquide ou de tout autre neutralisant gazeux ou liquide (soude, potasse, chaux, alcalis,...).

La température est maintenue entre 20 et 40°C, et préférentiellement à 30°C, grâce à un échangeur tubulaire 17 permettant de chauffer ou de refroidir le produit circulant dans la partie commune 18 des boucles externes 9,19, selon les besoins. Le rôle de la deuxième boucle 19 sera détaillé par la suite.

Le capteur 28 de mesure de teneur en biomasse permet de connaître la concentration de bactéries propioniques dans la cuve 6 de fermentation.

Il est à noter que la présence de la boucle de brassage 9 permet l'installation du capteur 28, un tel capteur ne pouvant être installé qu'en ligne. En effet, à l'heure actuelle, il n'existe pas de capteur de mesure pouvant être installé dans une cuve de fermentation pour effectuer des mesures de turbidité, ou autres...

La cuve 6 peut être de géométrie simple, ne nécessitant pas de renflements ou d'irrégularités nécessités par la présence de capteurs à l'intérieur de la cuve de fermentation, ce qui rend son prix de revient inférieur à celui d'une cuve comprenant des capteurs et un agitateur.

Un autre avantage réside dans la facilité de nettoyage des capteurs 14,15, par exemple dans le cas où une contamination est apparue. Une simple circulation d'un produit à pouvoir stérilisant sous pression dans le tube 18 contenant les capteurs 14,15 permet leur nettoyage.

Un avantage supplémentaire est la diminution des risques de contamination lors du fonctionnement de la cuve 6 de fermentation, en évitant les interventions directes dans la cuve 6 pour la prise de mesures.

On constate que la mise en oeuvre du procédé selon l'invention ne nécessite pas la présence d'un support pour les bactéries propioniques. Ceci permet des teneurs en biomasse plus élevées par unité de volume de cuve 6.

La circulation du substrat fermenté 8 selon le chemin A correspond à une première phase de fermentation qui sera explicitée par la suite.

Une seconde phase des cycles effectués selon la présente invention consiste à effectuer une extraction des bactéries 23 du mélange 21 à obtenir. Pour ce faire, la vanne 12 (fig.1) dirige le produit extrait de la cuve vers une deuxième boucle 19 externe appelée boucle d'extraction comprenant un module de filtration 22.

Le produit extrait de la cuve circule alors selon le chemin B. Le module de filtration 22 permet de séparer les bactéries 23 du mélange 21 à obtenir. Ce module peut être soit un module d'ultrafiltration, soit un module de microfiltration, en fonction du type de configuration de membrane (plane, à fibres creuses, tubulaire,..), de la membrane utilisée et du produit à obtenir.

Le mélange 21 est récupéré et les bactéries 23 sont recyclées en tout ou partie dans la cuve 6 de fermentation selon la position de la vanne 24. La vanne 13 est positionnée de telle sorte qu'il puisse y avoir un recyclage des bactéries 23 dans la cuve 6.

Les bactéries non recyclées sont récupérées à la sortie 26 du dispositif. Il est à noter qu'un remarquable avantage de la structure du dispositif de fermentation de l'invention réside dans le fait que le module de filtration 22 peut être nettoyé, décolmaté ou désinfecté pendant qu'il n'est pas en service, c'est à dire lors des périodes de croissance des cellules (circulation selon A), sans pour autant interrompre le processus de fermentation, du fait de la présence de la boucle de brassage et de la vanne commune de commutation 12.

Les vannes 12, 13, et 24 sont commandées pour que les phases de fermentation et d'extraction soient alternées pour correspondre à un procédé de fermentation semi-continue comme décrit ci-après.

La fermentation est réalisée sur une source carbonée. Cette source carbonée peut notamment être un dérivé laitier, tel que le lactosérum. Le lactosérum présente l'avantage de renfermer jusqu'à 14 % sur la matière sèche de protéines sériques dont la valorisation après purification est très intéressante.

C'est pourquoi, selon un mode de mise en oeuvre préférentiel de la présente invention, la fermentation propionique est réalisée sur un lactosérum déprotéiné 1 par ultrafiltration et faiblement supplémenté en azote 3 pour la survie des bactéries.

Avantageusement le perméat est enrichi 2, à raison de 0,5 à 1 % (poids/poids) par un complément 3 composé de 40 à 60 % de matière azotée, principalement sous forme d'acides aminés libres ou de peptides.

Il peut s'agir par exemple d'eaux de déchiquetis d'algues, d'extraits solubles d'eaux de lavage de germes de maïs, ou de tout autre supplément azoté (extraits ou hydrolysats de levures, hydrolysats de proteines animales ou végétales,...)

La composition globale est portée ci-dessous à titre d'exemple:

EP 0 452 240 A1

|  | eaux de maïs | eaux d'algues |
|---|---|---|
| Extrait sec | 95 % | 71,2 % |
| Matière grasse | 0 % | 0,1 % |
| Matière azotée | 49 % | 48,81 % |
| Cendres | 15 % | 4,2 % |
| pH | 4 | 7,2 |

Il est également avantageux d'utiliser un lactosérum obtenu par acidification lactique du lait. En effet, la présence de lactate dans le milieu de fermentation a un effet favorable sur la croissance des bactéries propioniques.

Comme pour tous les procédés de fermentation, le substrat doit être débarrassé des bactéries contaminantes. Cette opération de stérilisation 4 peut être obtenue de diverses manières, et notamment par filtration 4 sur membrane permettant la rétention des bactéries. Cela permet d'éviter les dénaturations de substrat obtenues lors des traitements thermiques traditionnels. Pour l'application effectuée, une membrane de microfiltration de seuil de coupure proche de 0,22 microns convient avantageusement.

La pompe 5 permet l'introduction du substrat stérilisé dans la cuve 6 de fermentation préalablement stérilisée.

Pour permettre un premier cycle de fermentation, un inoculum 7 est ajouté au substrat 8 présent dans la cuve 6. L'inoculum 7 est constitué de bactéries propioniques. Les bactéries propioniques ont pour fonction de transformer la source carbonée 8 (substrat) en acide propionique, acide acétique et $CO_2$.

Les souches de Propionibacterium les plus fréquemment utilisées pour la production d'acide propionique sont les Propionibacterium shermanii et freuden reicheii. Un mode de mise en oeuvre préférentiel de la présente invention consiste à utiliser des Propionibacterium thoenii pour la fermentation du substrat 8. Tout autre type de souches de Propionibacterium peut toutefois être utilisé pour la mise en oeuvre de l'invention.

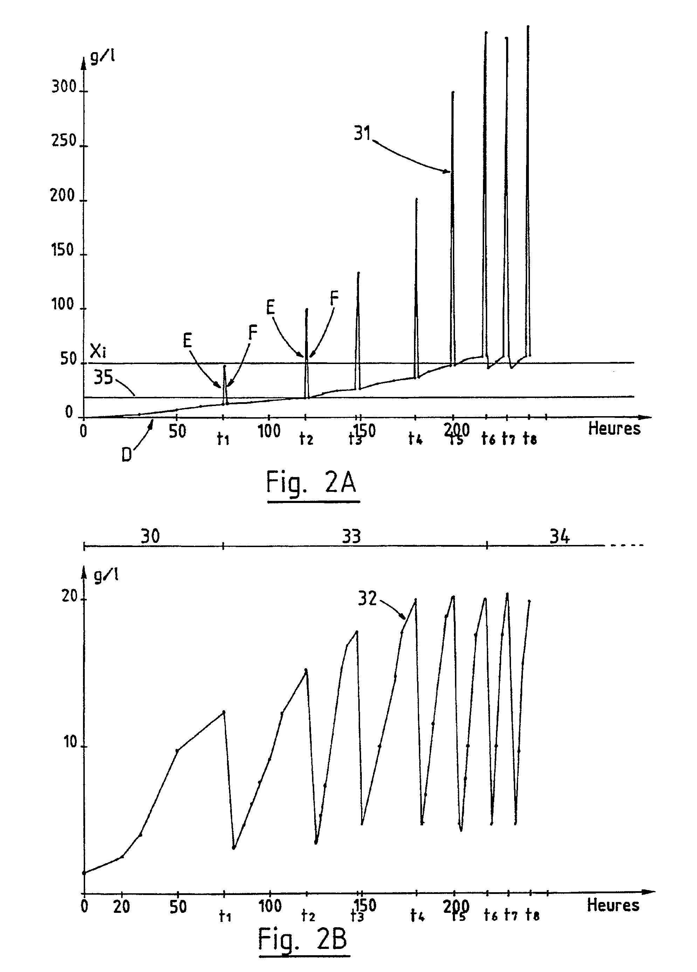

Les figures 2A et 2B permettent d'illustrer les caractéristiques essentielles du procédé de l'invention. Elles représentent les évolutions des concentrations de biomasse 31 (Fig.2A) et d'acide propionique 32 fabriqué (Fig.2B) relevées pendant une période de 250 heures permettant d'atteindre une quantité de biomasse ($X_l$) prédéterminée au delà de laquelle la productivité spécifique ne peut plus être maintenue.

Les deux courbes des figures 2A et 2B ont esseentiellement une valeur qualitative : elles correspondent à des essais ponctuels, réalisés dans des conditions non optimales. D'autres essais ont été réalisés, auxquels il sera fait référence plus loin, notamment en ce qui concerne les conditions quantitatives préférées, notamment les concentrations préférées d'apports de source carbonée, de biomasse etc..

La fermentation selon la présente invention comporte trois étapes successives, regroupables en deux périodes principales :
– une première période de croissance de la biomasse (30 + 33) ;
– une seconde période de fonctionnement en régime constant régulé (34).

La première période (30+33) peut être analysée sous forme de deux étapes successives. La première étape (30) correspond à l'inoculation du fermenteur qui servira une fois pour toutes pour l'ensemble des opérations de fermentation. En principe, on n'ajoutera en effet plus d'inoculum au milieu fermenté. Le fermenteur est inoculé par exemple de 3 à 15 % (volume), préférentiellement de 7 à 10 % et la fermentation peut alors commencer.

La première étape 30 est une fermentation discontinue. Celle-ci dure un temps $t_1$, proche de 70 heures dans l'exemple décrit. On remarque le long temps de latence D, caractéristique de la fermentation propionique.

La seconde étape (33) de la première période (30 + 33) commence à partir du temps $t_1$ et dure jusqu'au temps $t_6$.

Au bout du temps $t_1$, une partie du volume 8 présent dans la cuve 6 est retirée pour subir une opération d'extraction du mélange 21 à obtenir (acide propionique + acide acétique) avec recyclage des bactéries 23.

Cette extraction peut s'effectuer de différentes manières, notamment par:
– filtration (ultrafiltration ou microfiltration)

7

– centrifugation

– séparation chimique par solvant

L'extraction des constituants s'effectue de façon préférentielle par ultrafiltration 22.

Le filtrat 21 obtenu constitue le mélange contenant l'acide propionique et le rétentat 23 comprend les bactéries propioniques. Ce rétentat 23 est, dans chaque cycle de la seconde étape (33), entièrement recyclé dans la cuve 6 de fermentation pour augmenter la concentration de bactéries du milieu 8 de fermentation et servir d'inoculum pour la fermentation suivante. Ce recyclage est effectué à travers les vannes 13 et 24. Un volume de substrat neuf 27, équivalent au volume de mélange 21 récolté est alors introduit dans la cuve 6 de fermentation et le processus de fermentation peut reprendre.

Toute opération d'extraction provoque une augmentation importante (E) de la concentration de biomasse présente dans la cuve 6 de fermentation due au recyclage des bactéries propioniques. L'introduction de substrat neuf 27 provoque la diminution de cette concentration (F).

La répétition de ce processus peut être réalisée 3 à 5 fois, voire davantage, en fonction de la taille de la cuve 6 et du volume mort de l'installation. Ainsi, dans l'exemple de la figure 2, cette opération est répétée aux temps $t_2$, $t_3$, $t_4$, $t_5$, et $t_6$ correspondants chacun à une vidange partielle de la cuve 6 de fermentation puis à un recyclage total des bactéries retenues dans le filtre.

La deuxième étape (33) de la première période (30 + 33) est donc constituée d'une série de cycles successifs alternant des cycles d'augmentation de concentration bactérienne avec des cycles d'extraction des acides propionique et acétique fabriqués. On a ainsi en principe (bien que non obligatoirement): $t_1 > (t_2-t_1) > (t_3-t_2) > ... > (t_6-t_5)$.

Le recyclage total ou partiel des bactéries dans la cuve 6, est réalisé automatiquement par exemple à l'aide des mesures fournies par le capteur 28 de mesure de teneur en biomasse.

Le mélange 21 obtenu contenant l'acide propionique peut être stocké puis concentré. Avantageusement, le concentré peut être déshydraté en présence d'un support du type protéines, maltodextrines, amidon,...

L'acide propionique peut également être extrait du mélange et purifié pour une utilisation sous forme purifiée.

Au bout d'un certain nombre de cycles, six en l'occurence dans l'exemple des figures 2A et 2B, la concentration en biomasse atteint une valeur $X_l$ comprise entre 30 et 60 g/l et préférentiellement entre 45 et 55 g/l, au delà de laquelle la productivité spécifique ne peut plus être maintenue.

C'est alors qu'intervient la seconde période (34) (troisième étape) consistant à ne plus effectuer qu'un recyclage partiel des bactéries. Cela signifie qu'une partie 26 des bactéries 23 obtenues par séparation 22 n'est plus recyclée dans la cuve 6 de fermentation, de telle sorte que la concentration bactérienne est maintenue proche de la concentration $X_l$ optimale souhaitée. Cette concentration $X_l$ est avantageusement supérieure au seuil antibactérien. Au cours de cette seconde période (34), la durée de chaque cycle est sensiblement constante et très courte. Les cycles de fermentation/extraction sont, à partir du temps $t_6$, sensiblement constants $(t_7-t_6) \approx (t_8-t_7)$. Elle peut se prolonger indéfiniment, jusqu'à l'apparition d'une contamination ou d'une baisse d'activité de la souche.

Les bactéries non recyclées constituent une biomasse pouvant par exemple servir d'inoculum dans un autre dispositif selon la présente invention ou être utilisées comme décrit dans l'exemple 2 décrit par la suite.

Le passage de la phase de fermentation à la phase d'extraction au cours de chaque cycle est effectué selon une logique d'exploitation prédéterminée, notamment de façon à maximiser soit le rendement de fabrication du mélange 21 biologique, soit la vitesse de croissance des bactéries.

Plus précisément, le passage de ladite phase de fermentation à ladite phase d'extraction est avantageusement effectué lors de la réalisation d'au moins l'un des critères suivants :

– la teneur résiduelle en source carbonée est inférieure à 10 %, préférentiellement 5 % de la quantité de source carbonée introduite ;

– la concentration en acide propionique produit dépasse un seuil de 40 % environ de la concentration initiale en source carbonée.

Au début de chacun des cycles, l'apport de la source carbonée s'effectue selon une concentration prédéterminée par rapport au milieu liquide de réaction.

De façon préférentielle, la source carbonée est tout d'abord apportée en concentration moindre, de façon à ne pas surcharger la biomasse lors de sa phase initiale de croissance, ce qui pourrait avoir un effet inhibiteur.

Avantageusement, chaque quantité de source carbonée introduite, à chaque cycle, présente une concentration qui s'étage :

– pour au moins le début de ladite première période 30, 33, entre 35 et 45 g/l environ ;

– pour au moins ladite seconde période 34, entre 60 g/l et 110 g/l, préférentiellement 65 g/l à 80 g/l.

Lorsqu'on atteint une consommation de 90 à 95 % de la source carbonée introduite, la courbe 32 de fabrication d'acide propionique s'infléchit, et constitue un signe indicatif du moment de passage à la seconde phase d'extraction.

La mesure de la quantité d'acide propionique fabriqué peut également servir de seuil de changement de phase fermentation/extraction. Pour la souche de bactéries *Propionibacterium* utilisées pour les essais, il a été déterminé expérimentalement que 100 g de source carbonée introduite (lactose) fournissent environ 45 g d'acide au maximum. Cette souche bactérienne a été choisie parmi celles présentant le meilleur taux de conversion. Le changement de phase fermentation/extraction peut ainsi donc par exemple s'effectuer lorsqu'on constate que la concentration en acide propionique dépasse un seuil de 40 % environ de la concentration initiale en source carbonée, préférentiellement 42 %.

Pour suivre la concentration instantanée d'acide propionique et/ou acétique dans le milieu de fermentation, on peut utiliser par exemple des méthodes directes telles que des méthodes de chromatographie (de type HPLC). Ces méthodes sont toutefois lourdes, et difficilement réalisables en temps réel. De préférence, on utilisera plutôt le suivi de la courbe d'ajout de l'agent neutralisant 16 dans la cuve (voir fig.1). L'agent neutralisant est utilisé pour maintenir le pH du milieu de fermentation à une valeur sensiblement constante. Lorsque la production d'acide commence à stagner (infléchissement de la courbe 32), la vitesse d'introduction d'agent neutralisant diminue, et le basculement en phase d'extraction peut être effectué par seuillage.

Le processus de l'invention permet notamment de dépasser le plus tôt possible un seuil antibactérien. Ce seuil antibactérien est celui en deçà duquel le substrat fermenté peut être contaminé par des bactéries non propioniques de vitesse de croissance supérieure à celle des bactéries propioniques.

On a constaté qu'un seuil antibactérien est dépassé lorsqu'au moins un des critères suivants est vérifié:

– la concentration en bactéries de type *Propionibacterium* dépasse un certain seuil, de l'ordre de 40 g/l environ ;

– la concentration en acide propionique dans le milieu de culture dépasse un certain seuil, de l'ordre de 20 g/l environ.

Selon l'invention, l'un et/ou l'autre des seuils antibactériens, à savoir soit un seuil de concentration bactérienne, soit un seuil de concentration de l'acide propionique fabriqué, doivent être dépassés au moins pendant une portion de la phase d'extraction, et avantageusement également au moins pendant une portion de la phase de fermentation au cours d'au moins un des cycles.

Le seuil 35 représenté en figure 2A est avantageusement supérieur à 25 g/l, de préférence supérieur à 40 g/l. En effet, au-delà de cette concentration en biomasse, les bactéries propioniques sont moins vulnérables à la croissance de bactéries concurrentes.

L'une des caractéristiques avantageuses de l'invention est le fait que la concentration en biomasse atteint des taux très élevés, de façon fréquente, au cours de la phase d'extraction de chaque cycle. L'effet bactéricide envers les bactéries concurrentes est alors maximal.

De même, on pourrait tracer en figure 2B un seuil aux alentours de 20 g/l, correspondant à la concentration en acide propionique au-delà de laquelle on constate un effet antibactérien, comme détaillé plus bas. Dans l'essai réalisé et représenté en figure 2B, la production en acide propionique dépasse peu le seuil de 20 g/l, du fait des conditions de l'expérience. En effet, les tests correspondants ont été réalisés avec un apport de source carbonée faible. Ces conditions d'expérience ont permis d'obtenir les très bonnes valeurs de productivité volumique et de productivité spécifique présentées dans le tableau de l'exemple 2 ci-après.

D'autres essais ont également été menés, au cours desquels l'apport en source carbonée a été nettement supérieur : de l'ordre de 40 à 50 g/l en phase de croissance de la biomasse, et jusqu'à 90 g/l en phase stabilisée (période 34). La production d'acide propionique a alors pu atteindre des concentrations supérieures à 30 g/l, voire de l'ordre de 40 g/l. Le seuil antibactérien était alors largement franchi.

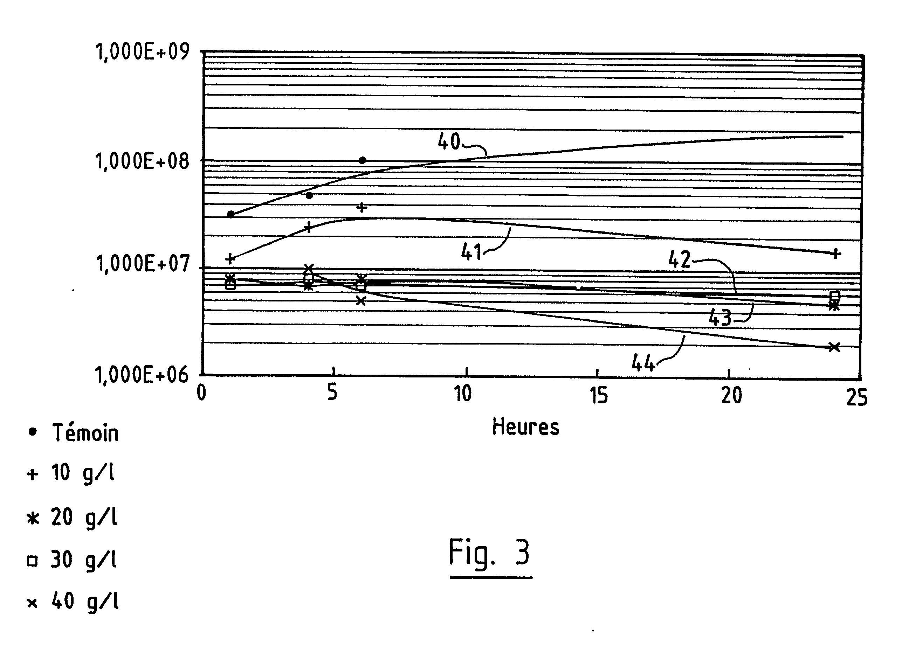

La figure 3 illustre le principe de lutte antibactérienne caractéristique du procédé de l'invention dans le cas où l'on utilise comme critère la concentration en acide propionique fabriqué. Elle correspond à des expériences de laboratoire étudiant l'évolution de la croissance d'un contaminant microbien suivant la concentration d'acide propionique dans un milieu de fermentation, en milieu à pH ajusté.

Le contaminant microbien est un *Streptococcus lactis*. Ce contaminant se retrouve fréquemment lors de cycles de fermentation de bactéries propioniques.

L'axe des ordonnées comporte une numération correspondant aux mesures de croissance effectuées des contaminants microbiens (nombre de cellules viables par ml.) et l'axe des abscisses est gradué en heures. La croissance des microbes *Streptococcus lactis* sans présence d'acide propionique est représentée par la courbe 40. Cette courbe montre une augmentation de croissance microbienne dans un milieu de culture neutre et correspond ainsi à un témoin.

La présence d'acide propionique dans des concentrations de 10, 20, 30 et 40 g/l respectivement permet de ralentir la croissance microbienne.

La courbe 41 montre un ralentissement de croissance microbienne pour une concentration d'acide propionique de 10 g/l environ et la quantité de bactéries concurrentes commence à diminuer après environ 6 à 7

9

heures.

A partir de 20 g/l (courbe 43), il se produit un effet bactériostatique immédiat c'est à dire que la concentration d'acide propionique est suffisante pour empêcher la croissance de contaminants (microbiens ou autres). Au bout de quelques heures, la concentration en biomasse utile devient bactéricide.

La courbe 42 correspond à une concentration de 30 g/l d'acide propionique environ : la concentration de 30 g/l permet d'obtenir des résultats proches de ceux obtenus avec une concentration de 20 g/l en réduisant le temps d'obtention de l'effet bactéricide.

La courbe 44 correspond à une concentration de 40g/l et permet d'obtenir un effet bactéricide immédiat.

Les exemples suivants décrivent la stérilisation et l'inoculation du substrat pour des volumes de fermenteur de 500 ml à 750 litres.

- Erlen de 500 ml et ballon de 6 litres.

Ces récipients sont remplis respectivement avec 250 ml et 3,5 litres. Le pH du milieu est ajusté à 7 avec de la soude 4N avant autoclavage mené à 115°C pendant 25 minutes pour l'erlen et à 120°C pendant 30 minutes pour le ballon.

Les cultures sont réalisées à 30°C sous agitation douce avec un ajustement quotidien du pH à 7. L'inoculum est apporté à raison de 10 % d'une culture de 50 à 70 heures.

- Fermenteur de 2 litres

Pour une stérilisation à chaud, le fermenteur contenant de 1 à 1,6 litre de milieu est autoclavé à 115°C pendant 30 minutes.

Pour une stérilisation à froid, le fermenteur contenant 1 litre d'eau est préalablement autoclavé à 115°C pendant 30 minutes puis est vidangé par mise sous pression d'azote stérile. Enfin le milieu microfiltré ou ultrafiltré pénètre dans le réacteur stérile.

L'ensemencement se fait à raison de 10 % (vol/vol) avec une culture de 40 à 60 heures.

Les conditions de fermentation sont 30°C, 150 RPM, pH 6,5 régulé par NaOH 4N ou $NH_4OH$ ou ammoniac gazeux ou toute autre base.

- Fermenteur de 750 litres

Le milieu est filtré sur membrane dont la paroi présente un diamètre de pores inférieur ou égal à 0,2 micron et est introduit dans un fermenteur préalablement désinfecté et stérilisé à 102°C pendant 30 minutes.

L'inoculum est une culture de 40 à 60 heures, préférentiellement de 45 heures, apporté à raison de 50 litres pour 500 litres de volume utile. Il a été préparé dans les mêmes conditions que le fermenteur 2 litres et stérilisé à chaud.

Les conditions de culture sont : 30°C, pH 6,5 régulé par une base concentrée.

Les trois exemples de fermentation suivants concernent le procédé de fermentation selon la présente invention :

## Exemple n° 1 culture en batch : Procédé de référence

Un fermenteur de 2 litres renfermant 1,1 litre d'ultrafiltrat de lactosérum acide à 5 % en matière sèche et complété par 1 % d'eaux de déchiquetage d'algues est stérilisé 30 min. à 115°C.

Le milieu est inoculé avec 100 ml d'une culture de 60 heures préalablement cultivée sur un milieu identique.

Au bout de 70 heures, tout le lactose a été consommé et 17,8 g/l d'acide propionique et 7,1 g/l d'acide acétique sont produits.

La productivité volumique maximale obtenue entre 25 et 40 heures atteint 0,40 g.l $^{-1}$ h $^{-1}$ pour l'acide propionique. Le rapport propionate/acétate est de 2,5 en fin de fermentation.

## Exemple n° 2 : culture semi-continue avec une souche pure.

Dans un fermenteur de 750 litres, un "batch" de 650 l est réalisé avec du perméat de lactosérum acide complémenté par 1 % d'eaux de déchiquetage d'algues et stérilisé par microfiltration sur des membranes de porosité 0,2 micron.

Le réacteur est ensemencé avec 50 litres d'une culture agée de 55 heures, préalablement cultivée sur un milieu identique, mais stérilisée à 115°C pendant 25 minutes. Après 60 heures de culture, la première séparation cellules/milieu a lieu. 550 litres du milieu contenant 18,8 g/l d'acide propionique et 8,5 g/l d'acide acétique sont collectés, et les cellules sont totalement recyclées dans le fermenteur. 550 litres de nouveau milieu contenant 1 % d'eaux d'algues sont alors rajoutés dans le réacteur après stérilisation à froid. Dès lors, cette opération est répétée toutes les 24 heures.

Après 300 heures, la concentration cellulaire atteint 50 g/l (exprimée en matière sèche). Dorénavant, à la fin de chaque recyclage, une fraction de la biomasse est éliminée par une purge au niveau rétentat, de façon à se maintenir entre 40 et 55 g/l.

Cette suspension concentrée de cellules peut alors être utilisée en tant que levain de fromagerie (pâtes pressées cuites type Emmental), en tant que probiotiques, qu'aliments pour animaux ou tout autre usage d'une biomasse de bactéries propionique sous la forme de suspension liquide ou après traitement de conservation (séchage, congélation, lyophilisation ou tout autre moyen de conservation de cellules microbiennes).

Au delà de 300 heures, la culture semi-continue peut se prolonger indéfiniment et ne sera interrompue qu'à l'apparition de contamination ou en cas de baisse de l'activité de la souche.

Les résutats obtenus sont portés dans le tableau suivant:

| heures de culture | concentration cellulaire g/l | Productivité volumique $g.^{l\text{-}1}\,h^{\text{-}1}$ | Productivité spécifique $g.\,g^{\text{-}1}$ | Propionate /acétate |
|---|---|---|---|---|
| 60 | 8 | 0,44 | 0,061 | 2,2 |
| 120 | 15 | 0,88 | 0,055 | 2,7 |
| 180 | 25 | 1,45 | 0,055 | 3,3 |
| 240 | 33 | 2,3 | 0,062 | 4,0 |
| 300 | 50 | 2,9 | 0,054 | 4,2 |
| 380 | 44 | 2,7 | 0,056 | 4,0 |

La concentration propionique passe de 6g/l à 18-20g/l entre chaque recyclage cellulaire. Le rapport propionate/acétate double après 300 heures de culture semi continue.

Par rapport à une fermentation par lot classique, la concentration cellulaire ainsi que la productivité volumique sont augmentées d'un facteur 6 sans constater pour autant une diminution de la productivité spécifique qui se maintient aux alentours de 0,055 g d'acide propionique/g cellules sèches/heure

Un exemple de composition de produit obtenu grâce au procédé mis en oeuvre par la présente invention est donnée à titre indicatif en g/100g :

| Extrait sec | 95,7 |
|---|---|
| Propionate de Calcium | 54,0 |
| Acétate de Calcium | 20,0 |
| Lactate de Calcium | 5,0 |
| Calcium | 18,0 |
| pH | 8,0 |
| Matière totale azotée (x 6,38) | 2,3 |

Exemple 3 : Culture semi-continue en culture mixte.

En accord avec la procédure décrite en exemple 2 la fermentation du perméat de lactoserum complémenté avec des extraits solubles d'eaux de lavage de germes de maïs est réalisée par coculture de *Propionibacterium thoenii* et de *Lactobacillus caseï*. La présence de *Lactobacillus caseï* montre une stimulation de l'activité du *Proponibacterium*. Il est entendu que d'autres bactéries lactiques pourraient être utilisées ou d'autres microorganismes susceptibles de stimuler le métabolisme de la bactérie propionique.

**Revendications**

1. Procédé de fermentation semi-continue d'un milieu de culture comprenant une source carbonée, notamment une substance à base de lactosérum, pour la fabrication industrielle d'un mélange (21) biologique à base d'acide propionique à l'aide d'une biomasse produite à partir d'un inoculum (7) de bactéries du type *Propionibacterium*,
caractérisé en ce qu'il consiste à réaliser une succession de cycles de fermentation/extraction, constitués chacun :
   – d'une première phase de fermentation au cours de laquelle ladite biomasse interagit en milieu fermé avec une nouvele quantité de ladite source carbonée ;
   – d'une seconde phase d'effraction périodique dudit mélange (21) biologique fabriqué, et de recyclage de la biomasse développée à partir de l'inoculum (7) pour la fabrication de mélange biologique (21) au cours de la phase de fermentation du cycle suivant ; et,
en ce qu'il comprend :
   – une première période formée d'une première succession de cycles fermentation/extraction, destinée à assurer la croissance de la biomasse constituée par lesdites bactéries du type *Propionibacterium* jusqu'à obtenir une quantité de biomasse prédéterminée $(X_l)$, la totalité de la biomasse étant recyclée d'un cycle à l'autre,
   – une seconde période formée d'une seconde succession de cycles fermentation/extraction, réalisés en maintenant ladite quantité de biomasse autour de ladite quantité prédéterminée $(X_l)$, en ne recyclant qu'une portion correspondante de la biomasse.

2. Procédé selon la revendication 1 caractérisé en ce que ladite quantité de biomasse $X_l$ est comprise entre 30 et 60 g/l, préférentiellement entre 45 et 55 g/l.

3. Procédé selon la revendication 1 caractérisé en ce que ladite phase d'extraction est réalisée à l'aide d'au moins l'une des méthodes suivantes :
- filtration (ultrafiltration ou microfiltration)
- centrifugation
- séparation chimique par solvant.

4. Procédé selon la revendication 1 caractérisé en ce que la concentration de source carbonée introduite à chaque cycle est compris :
- pour au moins le début de ladite première période (30,33), entre 35 et 45 g/l environ ;
- pour au moins ladite seconde période (34), entre 60 g/l et 110 g/l, préférentiellement 65 g/l à 80 g/l.

5. Procédé selon la revendication 1 caractérisé en ce que le passage de ladite phase de fermentation à ladite phase d'effraction est effectué de façon à optimiser l'un des critères appartenant au groupe comprenant la maximisation du rendement de fabrication dudit mélange (21) biologique à base d'acide propionique, et la maximisation de la vitesse de croissance desdites bactéries du type *Propionibacterium*.

6. Procédé selon la revendication 5 caractérisé en ce que le passage de ladite phase de fermentation à ladite phase d'extraction est effectué lors de la réalisation d'au moins l'un des critères suivants :
- la teneur résiduelle en source carbonée est inférieure à 10 %, préférentiellement 5 % de la quantité de source carbonée introduite ;
- la concentration en acide propionique produit dépasse un seuil de 40 % environ de la concentration initiale en source carbonée.

7. Procédé selon la revendication 6 caractérisé en ce qu'on détecte le dépassement du seuil de concentration en acide à partir de la surveillance de la vitesse d'ajout d'un produit neutralisant au mélange biologique pour maintenir le pH.

8. Procédé selon la revendication 1 caractérisé en ce que la concentration en bactéries et/ou la concentration en acide acétique et propionique dans le milieu de fermentation (8) dépasse un seuil antibactérien prédéterminé au moins au cours d'une portion de la phase d'extraction et/ou d'une portion de la phase de fermentation d'au moins un desdits cycles.

9. Procédé selon la revendication 8 caractérisé en ce que ledit seuil antibactérien (35) correspond à une concentration en biomasse et/ou en acide propionique dans le milieu fermenté présentant un effet bactériostatique et/ou bactéricide vis à vis des souches concurrentes contaminantes.

10. Procédé selon la revendication 8 caractérisé en ce que ledit seuil antibactérien est supérieur ou égal à 20 g/l environ de concentration en acide propionique et/ou acétique.

11. Procédé selon la revendication 8 caractérisé en ce que ledit seuil antibactérien est supérieur ou égal à environ 40 g/l de concentration en bactéries de type *Propionibacterium*.

12. Utilisation du procédé selon l'une quelconque des revendications 1 à 11 pour la fabrication de biomasse.

13. Dispositif de fermentation semi-continue caractérisé en ce qu'il est constitué d'une cuve de fermentation (6) comportant deux boucles externes (9,19) de circulation du substrat fermenté:
- une boucle (9) de brassage du substrat fermenté effectuant un prélèvement et une réintroduction dudit substrat fermenté dans ladite cuve (6) de fermentation,
- une boucle (19) d'effraction dudit mélange (21) biologique à base d'acide propionique fabriqué (21), incluant un module (22) de séparation mélange biologique fabriqué (21)/bactéries (23),
et en ce qu'il comprend des moyens d'aiguillage (12) sélectif de la circulation dudit substrat fermenté alternativement dans l'une (9) et l'autre (19) desdites boucles pilotés en fonction du procédé selon l'une quelconque des revendications 1 à 11

14. Dispositif selon la revendication 13 caractérisé en ce que ladite cuve (6) de fermentation est alimentée en substrat neuf (27) par des moyens de stérilisation (4) d'un milieu de culture (25) constitué par un ultrafiltrat (1) de lactosérum enrichi par un complément azoté (3).

15. Dispositif selon la revendication 13 caractérisé en ce que lesdites boucles de brassage (9) et d'extraction

13

(19) possèdent une partie commune (18) comprenant lesdits moyens d'aiguillage (12) sélectif de circulation dudit substrat fermenté, une pompe (10) ainsi que des moyens de mesure (14,15,28) de pH et/ou de température dudit substrat fermenté et/ou de teneur en biomasse.

16. Disposition selon la revendication 15 caractérisé en ce qu'il comprend des moyens d'introduction de produit neutralisant (16) gazeux ou liquide dans la cuve, pilotés par lesdits moyens (14) de mesure du pH afin de maintenir le pH du produit (8) présent dans ladite cuve (6) de fermentation à une valeur prédéterminée.

17. Dispositif selon la revendication 15 caractérisé en ce que ladite partie commune (18) comporte un échangeur thermique (17) permettant un chauffage ou un refroidissement dudit substrat fermenté (8).

Fig. 1

Fig. 2A

Fig. 2B

Fig. 3

● Témoin

+ 10 g/l

✱ 20 g/l

□ 30 g/l

× 40 g/l

EP 0 452 240 A1

Office européen
des brevets

**RAPPORT DE RECHERCHE EUROPEENNE**

Numero de la demande

EP 91 46 0022

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 338 098 (STARCOSA GmbH)<br>* Fig.; revendications 1-5; colonne 4, lignes 10-24 *<br>--- | 1,3,7,8 ,13-17 | C 12 M    1/12<br>C 12 P    7/52 |
| Y | US-A-3 472 765 (W.E. BUDD et al.)<br>* Figure 1; colonne 5, ligne 28 – colonne 6, ligne 38; colonne 4, lignes 27-33; colonne 3, ligne 61 – colonne 4, ligne 4 *<br>--- | 1,3,7,8 ,13-17 | |
| Y | WO-A-8 600 339 (NORDISK GENTOFTE A/S)<br>* Fig.; page 5, lignes 22-25 *<br>--- | 1,13 | |
| A | CHEMICAL ABSTRACTS, vol. 107, 1987, page 578, résumé no. 132555x, Columbus, Ohio, US; P. BLANC et al.: "Propionic acid fermentation: improvement of performances by coupling continuous fermentation and ultrafiltration", & BIOPROCESS ENG. 1987, 2(3), 137-9<br>* Résumé *<br>--- | 1 | |
| | | | **DOMAINES TECHNIQUES RECHERCHES (Int. Cl.5)** |
| Y | BIOTECHNOLOGY LETT., vol. 11, no. 3, 1989, pages 189-194; P. BLANC et al.: "Proprionic acid and biomass production using continuous ultrafiltration fermentation of whey"<br>* Page 190; page 191; page 193, conclusion *<br>----- | 1,3,7,8 ,13-17 | C 12 M<br>C 12 P |

Le présent rapport a été établi pour toutes les revendications

| Lieu de la recherche | Date d'achèvement de la recherche | Examinateur |
|---|---|---|
| LA HAYE | 04-07-1991 | COUCKE A.O.M. |